# EUROPEAN PATENT APPLICATION

(11) **EP 4 190 351 A1**
(43) Date of publication of application: **07.06.2023**
(21) Application number: 21852697.8
(22) Date of filing: 28.05.2021
(51) Int. Cl.: A61K 39/35, A61K 39/36, A61K 47/42, A61K 47/26, A61K 47/38, A61P 37/08

(54) **ALLERGEN PREPARATION**

(30) Priority: 03.08.2020 CN 202010766831
(71) Applicant: Glallergen Co., Ltd., Tianjin 301799 (CN)
(72) Inventor: BAI, Caiming, Tianjin 301799 (CN); ZHOU, Yan, Tianjin 301799 (CN); JIAN, Liping, Tianjin 301799 (CN); GUO, Tingting, Tianjin 301799 (CN); JIANG, Min, Tianjin 301799 (CN); JIA, Lixia, Tianjin 301799 (CN); WEI, Yonggang, Tianjin 301799 (CN)
(74) Representative: AWA Sweden AB
(86) International application number: PCT/CN2021/096889
(87) International publication number: WO 2022/028069

(57) **Abstract**

An application of collagen peptide in the preparation of an allergen preparation auxiliary material. For an allergen preparation, the auxiliary material thereof does not contain biological macromolecules, but contains the collagen peptide, avoiding the risk of potential allergies caused by the introduction of an inactive substance protein. The allergen preparation containing no biological macromolecule auxiliary materials can be stored stably for three years at room temperature. For allergic patients, the allergen preparation is a highly effective, stable and safe drug.

## Description

### FIELD

The present disclosure belongs to the field of pharmaceuticals, and particularly relates to an allergen preparation of which an auxiliary material contains collagen peptide.

### BACKGROUND

Allergic diseases are defined by the World Health Organization (WHO) as a major health problem in the world today. In 2010, WHO listed allergic diseases as the fourth most important chronic disease in the world. The total incidence of allergic diseases worldwide, including allergic asthma, is about 10-20%, therefore allergic diseases are common clinical diseases and frequently-occurring diseases. Currently, allergic diseases are getting worse. The main allergens that cause allergies are dust mites, pollen, house dust, molds, feathers, etc., and dust mites are one of the most powerful allergens distributed worldwide.

Asthma is one of the most common diseases in the world today, and patients suffering from asthma account for about 2-10% of the total population. There are about 50 million patients suffering from dust mite allergies worldwide, and dust mite allergy-induced asthma accounts for 10-30% of asthma. 45-80% of patients allergic to dust mites suffer from asthma, and dust mite allergies are one of the major factors in triggering asthma. There are 3 main species of dust mites that can cause allergic diseases in humans, i.e., *D. pteronyssinus* and *D. farinae* that belong to the genus *Dermatophagoides* of the subfamily *Dermatophagoidinae,* and *Euroglyphus maynei* belonging to the genus *Euroglyphus* of the subfamily *Pyroglyphinae. D. farinae* and *D. pteronyssinus* are much stronger than *Euroglyphus maynei* in terms of antigenic strength.

Allergic rhinitis is also a global health problem that is common throughout the world, with a global incidence of 10-25%. The number of patients is still on the rise. Allergic rhinitis seriously affects patients' daily life, study and work efficiency, and increases the load of medical treatment. Studies in China and other countries reveal that dust mites are the most important allergens in allergic rhinitis.

At present, treatment for allergic asthma and allergic rhinitis is generally divided into symptomatic treatment and specific immune treatment. Symptomatic treatment generally adopts a chemical drug such as an antihistamine drug and a glucocorticoid, but with the extension of treatment time, chemotherapy will be accompanied by a series of adverse reactions and side effects. For example, the resistance to the chemical drug will be developed, and the long-term application of a glucocorticoid will mildly depress the function of adrenal cortex. Since Noon and Freeman treated hay fever with a pollen extract in 1911, specific immune treatment of allergic diseases has been widely used for treating allergic diseases and has taken a leap in development. Allergen vaccines have gone through the development process of crude infusion, purified allergen vaccine, standardized allergen vaccine and allergen-like vaccine; in terms of dosage form, it has evolved from subcutaneous injection, sublingual drops to sublingual tablet.

Among the currently marketed injectable mite allergen vaccines, glycerol preparations are common in the American market, and mite allergen vaccines containing an aluminium hydroxide adjuvant are mainly prepared in European allergen vaccine manufacturers. Because the glycerol preparation mite allergen vaccine contains 50% glycerol, the maximum volume of each administration cannot exceed 0.2 mL, which makes it inconvenient to use. The glycerol preparation mite allergen vaccine is not as stable as a lyophilized vaccine, and it will increase patient's pain when used because it contains 50% glycerol. For the allergen vaccine containing an aluminium hydroxide adjuvant, although aluminium salts have an excellent track record of safety and adjuvant activity, they also have certain problems when applied. These problems include local reactions such as erythema, contact allergies, subcutaneous nodules, and granulomatous inflammation, and increased specific and general IgE antibody responses in experimental animals and humans (undesired antibody types in certain pathogenic cases such as an allergic reaction). In addition, it has been reported that aluminium salts are ineffective against certain antigens when used as adjuvants. It has also been reported that aluminium salts may cause experimental degenerative disorders of the central nervous system. Although there is no clinical evidence for proving the association of the vaccines containing aluminium with neurodegenerative disorders in humans such as Alzheimer's disease, the association between them has been presumed and widely debated. Unlike preventive vaccines, repeated administration is required during treatment of an allergic disease, the treatment cycle is as long as three years, resulting in a non-negligible problem of aluminium content accumulated in the human body. In terms of stability, the mite allergen vaccine containing an aluminium hydroxide adjuvant is not as stable as a lyophilized allergen vaccine.

Allergen sublingual administration began in 1990s, and compared with allergen injection administration, sublingual administration causes fewer side effects and has better dependence for patients. At the beginning of this century, allergen preparations for sublingual administration have been developed from sublingual drops to sublingual tablets. Compared with the allergen drops, the allergen sublingual tablets have great advantages. Firstly, the sublingual tablets can be stored at room temperature, so they are convenient to transport and carry. Secondly, the sublingual tablets are generally administered as a single dose, so the dosage administration is accurate. Thirdly, the dependence of patients on the sublingual tablets is better.

After the allergen sublingual tablets are stored at room temperature for 36 months, allergen active proteins are stable. In the current technology, a protein is added to a tablet excipient auxiliary material solution as an excipient auxiliary material solution, and commonly used proteins are gelatin proteins such as fish gelatin.

At present, the patent No. 200380107865.8 provides a formula of an allergen sublingual oral tablet, using mannitol and fish gelatin, or mannitol and starch as a matrix solution, and after the dosage form is stored at 25°C and the humidity of 60% for 3 months, the loss of the allergen content in the dosage form is less than 50% of the original allergen content.

### SUMMARY

In an aspect, the present application provides use of collagen peptide in the preparation of an allergen preparation auxiliary material.

Collagen peptide is an important functional protein, which is a product hydrolyzed from collagen and has the characteristics of low molecular weight and easy absorption. At present, collage peptide is used as a health-care product for improving the function of human body.

In an aspect, the present application also provides an auxiliary material composition, which contains collagen peptide.

In some embodiments, the auxiliary material composition also contains mannitol.

In some embodiments, the auxiliary material composition also contains cellulose.

In some embodiments, the cellulose is selected from one or two of hydroxyethyl cellulose and hydroxypropyl methyl cellulose.

In some embodiments, the auxiliary material composition contains the following substances in parts by weight:

| | |
|---|---|
| mannitol | 2-8 parts, preferably 2-5 parts; |
| collagen peptide | 2-10 parts, preferably 3-8 parts; and |
| cellulose | 0.2-2 parts, preferably 0.4-1 part. |

In some embodiments, the auxiliary material composition is a solution.

In some embodiments, the concentration of each substance in the auxiliary material composition solution is as follows:

| | |
|---|---|
| mannitol | 20-80 g/L, preferably 20-50 g/L; |
| collagen peptide | 20-100 g/L, preferably 30-80 g/L; and |
| cellulose | 2-20 g/L, preferably 4-10 g/L. |

In some embodiments, the collagen peptide is selected from one or more of fish collagen peptide, bovine bone collagen peptide, bovine skin collagen peptide, and pig skin collagen peptide.

In some embodiments, the collagen peptide is selected from one or two of fish collagen peptide and bovine bone collagen peptide.

In some embodiments, the collagen peptide is selected from fish collagen peptide.

In some embodiments, the fish collagen peptide is selected from cold water fish collagen peptide. An optional cold fish is, for example, *Oncorhynchus, Thunnus albacares, Gadus, Anguilla, Pleuronichthys cornutus, Acipenser sinensis, Perca fluviatilis* or *Oreochromis mossambicus.*

In some embodiments, the collagen peptide has a molecular weight of 3000-10000 Da.

In some embodiments, the collagen peptide has a molecular weight of 3000-9000 Da.

In some embodiments, the collagen peptide has a molecular weight of 3000-8000 Da.

In some embodiments, the collagen peptide has a molecular weight of 3000-7000 Da.

In some embodiments, the collagen peptide has a molecular weight of 3000-6000 Da.

In some embodiments, the collagen peptide has a molecular weight of 3000-5000 Da.

In an aspect, the present disclosure also provides use of the auxiliary material composition in the preparation of an allergen preparation.

In an aspect, the present disclosure also provides an allergen preparation semi-finished product, and an auxiliary material of the semi-finished product contains collagen peptide.

In some embodiments, the allergen semi-finished product contains the auxiliary material composition solution.

In some embodiments, the content of allergen is 0.05-2.5 mg, preferably 0.25-1 mg, more preferably 5-50 ug.

In an aspect, the present disclosure also provides an allergen preparation, and an auxiliary material of the preparation contains collagen peptide.

In some embodiments, a dosage form of the allergen preparation is selected from lyophilized powder, injection, tablet, capsule, drop, and patch.

In some embodiments, the dosage form of the allergen preparation is a tablet, more preferably a sublingual oral tablet.

In some embodiments, after the tablet is stored at 40°C and the relative humidity of 75% for 6 months, the loss of the allergen content in the tablet is less than 25% of labelled content; and further preferably, the loss of the allergen content in the tablet is less than 15% of the labelled content.

In some embodiments, after the tablet is stored at 25°C and the relative humidity of 75% for 36 months, the loss of the allergen content in the dosage form is less than 25% of the labelled content; and further preferably, the loss of the allergen content in the tablet is less than 15% of the labelled content.

In some embodiments, the allergen is selected from one or more of a house dust mite allergen, a pollen allergen, a mold allergen, an insect allergen, an animal dander allergen, and a food allergen.

In some embodiments, the house dust mite allergen is selected from one or two of *Dermatophagoides pteronyssinus* and *Dermatophagoides farinae.*

In some embodiments, the house dust mite allergen is one or more of allergenic proteins Derp1, Derf1, Derp2, and Derf2 that are separated from natural extracts of *Dermatophagoides pteronyssinus* and *Dermatophagoides farinae* or are recombinantly expressed.

In some embodiments, the pollen allergen is selected from one or more of a weed pollen allergen and a tree pollen allergen.

In some embodiments, the weed pollen allergen is selected from one or more of *Artemisia sieversiana* pollen, *Artemisia vulgaris* pollen, *Artemisia annua* pollen, *Ambrosia artemisiifolia* pollen, *Ambrosia trifida* pollen, *Humulus* pollen, *Chenopodium album* pollen, *Xanthium strumarium* pollen, and *Bassia scoparia* pollen.

In some embodiments, the tree pollen allergen is selected from one or more of *Populus* pollen, *Salix* pollen, *Ulmus* pollen, *Juniperus chinensis* pollen, *Fraxinus pennsylvanica* pollen, *Platanus* pollen, *Betula* pollen, *Cryptomeria* pollen, and *Acer* pollen; and more preferably, the *Platanus* pollen is selected from one or more of *Platanus orientalis* pollen and *Platanus acerifolia* pollen.

In some embodiments, the animal dander allergen is selected from one or two of cat dander and dog dander.

In some embodiments, the allergy is an IgE-mediated allergy.

In some embodiments, the allergy is a specific IgE-mediated allergy.

In some embodiments, the allergy includes one or more of allergic urticaria, allergic conjunctivitis, allergic rhinitis, and allergic asthma.

In an aspect, the present disclosure also provides a preparation method of an allergen preparation, which includes the following steps:
filling a semi-finished solution of an auxiliary material solution containing collagen peptide and an allergen stock solution into blisters, and transferring the blisters to a lyophilizer for lyophilization; the concentration of each substance in the auxiliary material solution being as follows:

| | |
|---|---|
| mannitol | 20-80 g/L, preferably 20-50 g/L; |
| collagen peptide | 20-100 g/L, preferably 30-80 g/L; and |
| cellulose | 2-20 g/L, preferably 4-10 g/L. |

In some embodiments, the semi-finished solution is prepared by the following method: mixing the auxiliary material solution containing collagen peptide with the allergen stock solution containing an allergen extract.

In some embodiments, the preparation method includes the following steps:
1) preparation of an allergen stock solution: purifying and degreasing an allergen, adding to a disodium hydrogen phosphate-sodium dihydrogen phosphate buffer (50-200 mM, PH=7.8-8.2) in a feeding ratio of 1: (25-50), extracting at 2-8°C for 4-24 h, centrifuging an extract obtained after extraction at 2-8°C and 6000-10000 rpm for 2-50 min, collecting and filtering a supernatant obtained after centrifugation to obtain an allergen extract solution, performing constant-volume ultrafiltration on the allergen extract solution obtained after clarification and filtration with purified water and a 3-5 KD membrane, and ultra-filtering and concentrating to the concentration of the allergen extract being not less than 0.75 mg/mL;
2) preparation of an auxiliary material solution: weighing and dissolving mannitol in water, weighing and adding collagen peptide to the mannitol solution, weighing and dissolving cellulose in the above mannitol-collagen peptide solution, and adding water to fix the volume;
3) preparation of an allergen semi-finished product: uniformly mixing the allergen stock solution prepared at step 1) with the auxiliary material solution prepared at step 2) in a volume ratio;
4) filling: filling the semi-finished solution prepared at step 3) into blisters;
5) lyophilization: transferring the blisters prepared at step 4) to a lyophilizer for lyophilization, preferably, placing the blisters prepared at step 4) in a liquid nitrogen tunnel or a liquid nitrogen freezer for 5-10 min, and transferring to a lyophilizer for lyophilization; and
6) sealing: after the lyophilization at step 5) is completed, taking out the blisters, covering with a film at 160-200°C, indenting, and cutting into unit blocks.

In some embodiments, at step 1), the supernatant obtained after centrifugation is filtered with a microfiltration membrane.

In some embodiments, the supernatant obtained after centrifugation is filtered with 0.8 µm, 0.45 µm, and 0.22 µm microfiltration membranes in sequence.

In some embodiments, the allergen extract obtained after clarification and filtration is subjected to constant-volume ultrafiltration with purified water and the 3-5 KD membrane to a 5-time volume.

In some embodiments, at step 4), 0.2-0.35 mL of allergen semi-finished solution prepared at step 3) is subpackaged into each blister.

In an aspect, the present disclosure also provides an allergen preparation prepared by the preparation method.

Lyophilized orally disintegrating tablet is a pharmaceutical dosage form that disintegrates rapidly in the sublingual mucosa. Due to its characteristics, it is necessary to select an appropriate auxiliary material formula for a good qualified orally disintegrating tablet to ensure that the dosage form has a loose network structure, can quickly disintegrate under the tongue, and has sufficient hardness, such that degranulation, powder loss, and release of residues to the environment will not occur during storage, transportation and use. For an allergen sublingual oral tablet, an appropriate auxiliary material is selected to ensure that the dosage form quickly disintegrates under the tongue. However, under the premise of ensuring the solid hardness of the dosage form, higher requirements are raised. Because the allergen is a factor of allergy, there are higher requirements for the allergen residue remaining in the blister.

A typical tablet excipient provides a skeleton for the tablet after lyophilization. Common skeleton excipients are some high-molecular polymers such as gelatin, Arabic gum, hydroxymethyl cellulose, pectin, dextrin, and various alginates. However, biological macromolecules are proteins and polysaccharides, both of which are sources of allergens.

### BRIEF DESCRIPTION OF THE DRAWINGS

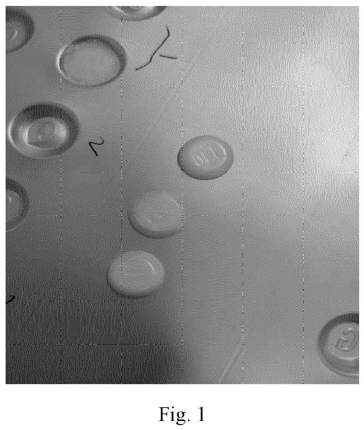

Fig. 1 is an external view of a dust mite tablet prepared in Example 3.

### DETAILED DESCRIPTION

The technical solutions of the present disclosure will be further described below with reference to specific examples, but the specific examples are not intended to limit the scope of protection of the present disclosure. Some nonessential modifications and adjustments made by others based on the concept of the present disclosure shall still fall within the scope of protection of the present disclosure.

In the present application, the term "allergen" refers to any naturally occurring protein or protein mixture, which has been reported to induce allergic reactions, i.e., IgE-mediated reactions, upon repeated exposure in individuals. Examples of naturally occurring allergens include pollen allergens (tree, weed, herb and grass pollen allergens), mite allergens (derived from, for example, *Dermatophagoides pteronyssinus* and *Dermatophagoides farinae*), insect allergens (inhaled allergens and allergens derived from saliva and venom), animal allergens derived from, for example, saliva, hair and dander of dogs, cats, horses, rats, mice, etc., fungi allergens, and food allergens. is the allergen may be used in the form of an allergen extract, a purified allergen, a modified allergen or recombinant allergen or recombinant mutant allergen, any allergen fragment containing more than 30 amino acids or any combination thereof.

The term "allergen extract" refers to an extract obtained by extracting a biological allergen source material, generally as follows: "Allergenic extracts", H. Ipsen et al., Chapter 20, "Allergy, principle and practice" (Ed. S. Manning) 1993, Mosby-Year Book, St. Louis. Such an extract can be obtained by water-extraction of a water-soluble material, followed by purification steps, such as filtration, to obtain a solution, i.e., an extract. Then, the extract can be further purified and/or treated, such as lyophilized, to remove substantially all of the water. Usually, an allergen extract includes a mixture of proteins and other molecules. Allergen proteins are often classified as major allergens, intermediate allergens, minor allergens, or unclassified. An allergen extract usually includes not only major allergens but also minor allergens. Major allergens usually account for about 5-15%, more usually about 10%, of a typical allergen extract. Classification of allergens is based on an evaluation of the clinical importance of specific allergens, as given below. Examples of important major allergens found in extracts include *Dermatophagoides pteronyssinus* 1 and 2 allergens (e.g., Derp1 and Derp2), tree pollen allergen 1 (Betv1), and *Ambrosia* pollen 1 and 2 allergens (Amba1 and Amba2). Generally, people with allergies are allergic and react to one or more major allergens, and further may be allergic and react to minor allergens.

In the present application, the amount of allergen extract refers to the dry matter content of allergen extract.

In the present application, the term "biological allergen source material" refers to any biological material containing one or more allergens. For example, the biological allergen source material may be a purified mite body (PMB) or whole mite culture (WMC), degreased or non-degreased pollen derived from, for example, grasses, herbs, weeds and trees, animal hair, dander or fur, fungal mycelium or spores, an insect body, venom or saliva, and food.

### Example 1 Preparation and evaluation of auxiliary material solutions

Auxiliary material solutions were prepared according to formulas of auxiliary material solutions in Table 1-1, and dust mite sublingual oral tablet semi-finished solutions were prepared according to Table 1-2.

### 1.1 Sources of auxiliary material components

Mannitol: Merck
Fish gelatin: Sigma
Gelatin: Guangzhou Rousselot Pharmaceutical Co., Ltd.

Corn starch: Shandong Liaocheng Ahua Pharmaceutical Co., Ltd.

Fish collagen peptide (5000 Da): Guangzhou Rousselot Pharmaceutical Co., Ltd.

Fish collagen peptide (3000 Da): Guangzhou Rousselot Pharmaceutical Co., Ltd.

Fish collagen peptide (2000 Da): Guangzhou Rousselot Pharmaceutical Co., Ltd.

Fish collagen peptide (1000 Da): Guangzhou Rousselot Pharmaceutical Co., Ltd.

In examples herein, all the fish collagen peptides were cold water fish collagen peptides, and the fish collagen peptides of different molecular weights were derived from the cold water fish *Oreochromis mossambicus.*

Bovine bone collagen peptide (300 Da): Guangzhou Rousselot Pharmaceutical Co., Ltd.

Bovine skin collagen peptide (3000 Da): Guangzhou Rousselot Pharmaceutical Co., Ltd.

Hydroxyethyl cellulose: Beijing Wise & Well Technology Co., Ltd.

Cyclodextrin: Beijing Wise & Well Technology Co., Ltd.

rHSA: North China Pharmaceutical Co., Ltd.

Hydroxypropyl methyl cellulose: Beijing Wise & Well Technology Co., Ltd.

Maltodextrin: Changling Jilong Biological Pharmaceutical Co., Ltd.

### 1.2 Preparation of low molecular weight maltodextrin

Maltodextrin was dissolved in water, after a clear solution was obtained, the clear solution was added to frozen absolute ethanol in a volume ratio of 1: 9, placed for 2 h, and centrifuged at 8000 rpm/min and 2-8°C for 15 min, a supernatant was discarded, precipitates were redissolved in water, the redissolved precipitates were clarified and filtered, put in a G50 gel column, and washed with water, a liquid of the IV peak was collected, and the collected liquid was lyophilized to obtain low molecular weight maltodextrin. The molecular weight of the low molecular weight maltodextrin was measured by exclusion chromatography.

### 1.3 Preparation of auxiliary material solutions

Components of each formula in Table 1-1 were weighed in sequence and dissolved in water, and finally purified water was added to fix the volume.

### 1.4 Preparation of allergen tablets

1) preparation of an allergen stock solution: cultured, harvested, inactivated, purified and degreased *Dermatophagoides pteronyssinus* and *Dermatophagoides farinae* raw materials were respectively added to a disodium hydrogen phosphate-sodium dihydrogen phosphate buffer (200 mM, PH=7.8-8.2) in a feeding ratio of 1: 50, the solution was extracted at 2-8°C for 20 h; an extract obtained after extraction was centrifuged at 2-8°C and 8000 rpm for 15 min, a supernatant obtained after centrifugation was filtered with 0.8 µm, 0.45 µm, 0.22 µm microfiltration membranes in sequence to obtain an allergen extract; the allergen extract obtained after clarification and filtration was subjected to constant-volume ultrafiltration with purified water and a 3 KD membrane to a 5-time volume, and the allergen extract was ultra-filtered and concentrated to the concentration of *Dermatophagoides pteronyssinus* extract in the *Dermatophagoides pteronyssinus* stock solution being 2 mg/mL or the concentration of *Dermatophagoides farinae* extract in the *Dermatophagoides farinae* stock solution being 8 mg/mL to obtain an allergen stock solution.
2) The auxiliary material solution prepared at step 1.3 was mixed with the allergen stock solution in the following manner to obtain an allergen semi-finished solution containing a required dose of allergen extract.
   Equal volumes of *Dermatophagoides pteronyssinus* stock solution and *Dermatophagoides farinae* stock solution that were prepared at step 1) were uniformly mixed, such that the concentrations of the allergen extracts in the two stock solutions in the obtained mixture (uniformly mixed dust mite solution) were respectively halved, that is, the concentration of the *Dermatophagoides pteronyssinus* extract was 1 mg/mL and the concentration of the *Dermatophagoides farinae* extract was 4 mg/mL. The above uniformly mixed dust mite solution was uniformly mixed with the auxiliary material solution in a volume ratio of 1: 1, such that the concentrations of the allergen extracts in the semi-finished solution intermediate were respectively halved again, that is, the concentration of the *Dermatophagoides pteronyssinus* extract was 0.5 mg/mL and the concentration of the *Dermatophagoides farinae* extract was 2 mg/mL, and the semi-finished solution intermediate was diluted with the auxiliary material solution with the equal volume to obtain a dust mite semi-finished solution in which the concentration of the *Dermatophagoides pteronyssinus* extract was 0.25 mg/mL and the concentration of the *Dermatophagoides farinae* extract was 1 mg/mL.
3) Filling: the semi-finished solution prepared at step 2) was filled into blisters, and 0.2-0.35 mL of allergen semi-finished solution prepared at step 2) was subpackaged into each blister.
4) Lyophilization: the blisters prepared at step 3) were transferred to a lyophilizer for lyophilization; and specifically, the blisters prepared at step 3) were placed in a liquid nitrogen tunnel or a liquid nitrogen freezer for 5-10 min, and then transferred to a lyophilizer for lyophilization.
5) Sealing: after the lyophilization at step 4) was completed, the blisters were taken out, covered with a film at 160-200°C, indented, and cut into unit blocks.

Allergen tablets 1-21 were respectively prepared from auxiliary materials 1-21 in Table 1-1.

In the selection of auxiliary material components, the viscosity of the semi-finished solution should be sufficient to be able to be subpackaged into blisters.

In this example, the formulas of the preparation auxiliary materials were evaluated according to whether the semi-finished solution was fillable, the shape of the tablet, residues in blisters and the disintegration speed.

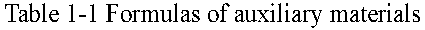

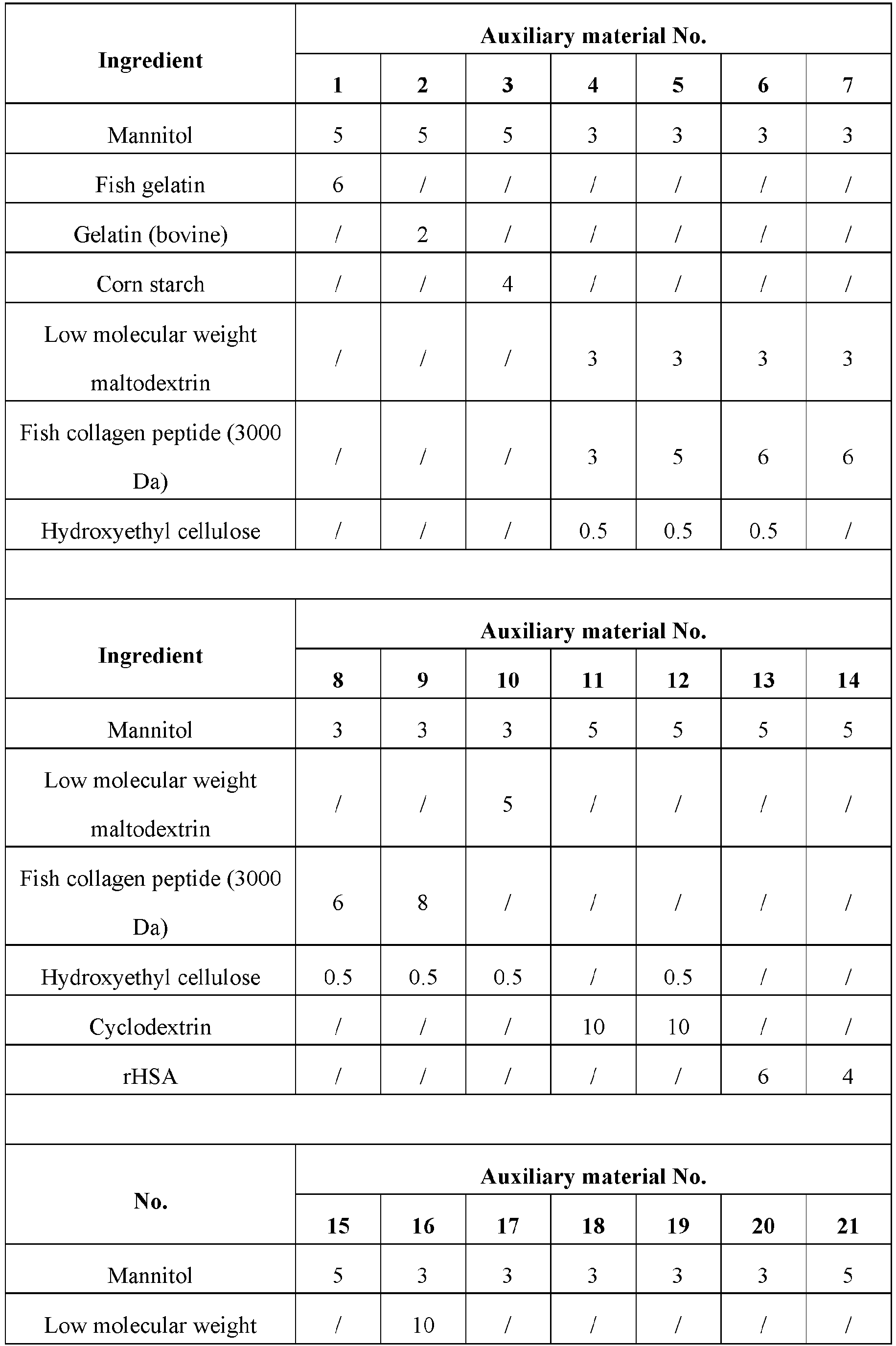

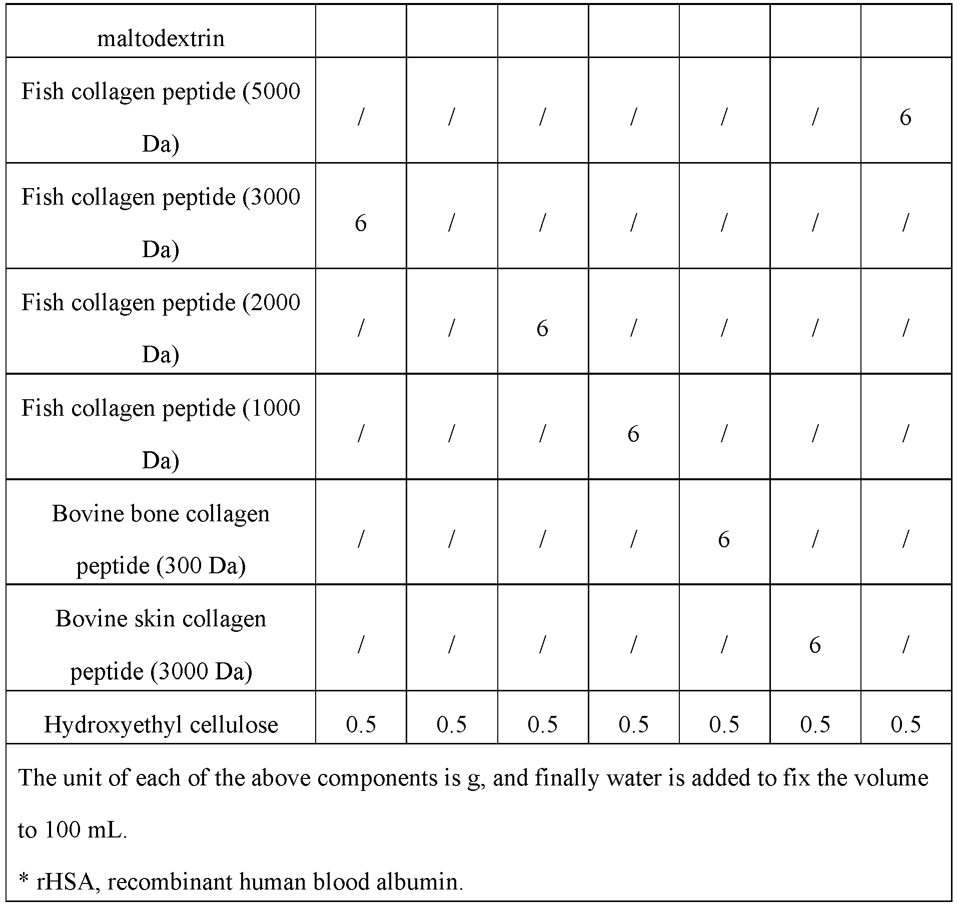

**Table 1-2 Evaluation results of formulas of allergen tablets**

| Tablet No. | Viscosity of liquid | Appearance | Residue in blister | Disintegration speed |
|---|---|---|---|---|
| 1 | fillable | intact shape, and good hardness | no residual powder in blisters | 30 s |
| 2 | fillable | bad shape | no residual powder in blisters | 90 s |
| 3 | fillable | intact shape, and acceptable hardness | no residual powder in blisters | 30 s |
| 4 | fillable | incomplete edges | no residual powder in | 30 s |
| | | | blisters | |
| 5 | fillable | intact shape, and acceptable hardness | no residual powder in blisters | 15 s |
| 6 | fillable | intact shape, and acceptable hardness | no residual powder in blisters | 10 s |
| 7 | fillable | intact shape, and acceptable hardness | a small amount of residual powder in blisters | 15 s |
| 8 | fillable | intact shape, acceptable hardness, and slightly rough edges | no residual powder in blisters | 10 s |
| 9 | fillable | intact shape, acceptable hardness, and slightly rough edges | no residual powder in blisters | 10 s |
| 10 | fillable | incomplete shape, and acceptable hardness | no residual powder in blisters | 60 s |
| 11 | fillable | incomplete shape, and poor hardness | a large amount of residual powder in blisters | 60 s |
| 12 | fillable | incomplete shape, and poor hardness | a large amount of residual powder in blisters | 60 s |
| 13 | fillable | incomplete shape, and poor hardness | a large amount of residual powder in blisters | 60 s |
| 14 | fillable | incomplete shape, and poor hardness | a large amount of residual powder in blisters | 60 s |
| 15 | fillable | intact shape, and acceptable hardness | no residual powder in blisters | 5 s |
| 16 | fillable | intact shape, and acceptable hardness | no residual powder in blisters | 5 s |
| 17 | fillable | incomplete shape, and poor hardness | a large amount of residual powder in blisters | 20 s |
| 18 | fillable | incomplete shape, and poor hardness | a large amount of residual powder in blisters | 10 s |
| 19 | fillable | incomplete shape, and poor hardness | a large amount of residual powder in blisters | 10 s |
| 20 | fillable | incomplete shape, and poor hardness | a large amount of residual powder in blisters | 30 s |
| 21 | fillable | intact shape, and acceptable hardness | no residual powder in blisters | 10 s |

It can be seen from the results in Table 1-2 that the conventional common auxiliary materials adopting macromolecular auxiliary material formulas, including tablets containing the auxiliary material compositions of fish gelatin (auxiliary material No. 1) and corn starch (auxiliary material No. 3), have good indicators, but their allergenicity cannot be ignored. However, the preparation containing gelatin (auxiliary material No. 2) has very unsatisfactory indicators in terms of appearance and residue in blister.

In addition to collagen peptide, in a case that other non-macromolecular compounds (hydroxyethyl cellulose, cyclodextrin, and rHSA) were selected as auxiliary material formulas to replace gelatin/corn starch, the prepared tablets (11-14) have unsatisfactory indicators in terms of appearance and residue in blister.

However, in a case that collagen peptide is selected, especially collagen peptide of appropriate molecular weight is selected, it has a better effect compared to other conventional non-macromolecular auxiliary materials.

A preferred molecular weight range of the collagen peptide is 3000-8000 Da, especially preferably 3000-5000 Da.

The inventors have also found that collagen peptides derived from different sources, even in similar molecular weight ranges, will not necessarily have the same effect. Particularly, it is found that fish collagen peptide has a significantly better effect compared to collagen peptides derived from other sources. In the actual development process, the inventors have also tested pig skin collagen peptide, and its results are similar to those of bovine skin or bovine bone collagen peptide.

The preparation containing the composition of fish collagen peptide have satisfactory indicators, and the disintegration speed of formula 15 can reach 5 s.

### Example 2 Study on the allergenicity of the allergen preparation auxiliary materials

In this example, the allergenicity of each formula of the allergen preparation auxiliary material was evaluated.

Auxiliary material solutions were prepared according to formulas of preparations in Table 2-1.

Other steps were the same as those in Example 1, and in the semi-finished solution, the concentration of the *Dermatophagoides pteronyssinus* extract was 0.25 mg/mL and the concentration of the *Dermatophagoides farinae* extract was 1 mg/mL.

**Table 2-1 Formulas of auxiliary material solutions**

| **Ingredient** | **Unit** | **Formula 1** | **Formula 2** | **Formula 3** |
|---|---|---|---|---|
| Mannitol | g | 5 | 5.08 | 5.08 |
| Fish collagen peptide (5000 Da) | g | 7.5 | -- | -- |
| Fish gelatin | g | -- | 6 | -- |
| Corn starch | g | -- | -- | 4 |
| Hydroxyethyl cellulose | g | 0.5 | -- | -- |
| Add water to fix the volume to | mL | 100 | 100 | 100 |

Dog models with sea fish and corn allergies were constructed. The dog models with sea fish allergies were divided into 2 groups, the dog models with corn allergies were divided into 2 groups, two control groups of dogs without sea fish, dust mite and corn allergies were set, 8 dogs per group, and all the dogs were respectively subjected to sublingual administration according to Table 2-2.

**Table 2-2 Animal experiment**

| Group | Group 1 | Group 2 | Group 3 | Group 4 | Control 1 | Control 2 |
|---|---|---|---|---|---|---|
| Model | Sea fish allergy | | Corn allergy | | Without sea fish, corn and dust mite | |
| | | | | | allergies | |

| Type of drug | Formula 1 | Formula 2 | Formula 1 | Formula 3 | Formula 1 | Formula 3 |
|---|---|---|---|---|---|---|
| Administration | one tablet per day via the sublingual mucosa, for 7 consecutive days | | | | | |

Adverse reactions of each dog after administration were recorded daily, including sneezing, watery eyes, hyperactivity, and redness and swelling of the conjunctiva of the dog. Results are shown in Table 2-3.

**Table 2-3 Reactions of animals after administration**

| Group | Group 1 | Group 2 | Group 3 | Group 4 | Control 1 | Control 2 |
|---|---|---|---|---|---|---|
| | Animals with allergic reactions (individuals) | | | | | |
| Day 1 of administration | 0 | 4 | 0 | 3 | 0 | 0 |
| Day 2 of administration | 0 | 3 | 0 | 3 | 0 | 0 |
| Day 3 of administration | 0 | 3 | 0 | 2 | 0 | 0 |
| Day 4 of administration | 0 | 3 | 0 | 2 | 0 | 0 |
| Day 5 of administration | 0 | 3 | 0 | 2 | 0 | 0 |
| Day 6 of administration | 0 | 3 | 0 | 2 | 0 | 0 |
| Day 7 of administration | 0 | 3 | 0 | 2 | 0 | 0 |

It can be known from the results shown in Table 2-3 that after the allergen sublingual oral tablets containing the fish collagen peptide composition of the present disclosure are administered to the animal models with sea fish or starch allergies, no allergic adverse reactions develop, which indicate that the auxiliary material formula will not introduce allergens.

### Example 3 Dust mite allergen preparations with an auxiliary material containing collagen peptide

### 3.1 Preparation

### 3.1.1 Preparation of an auxiliary material solution

According to a formula in Table 3-1, mannitol was weighed and dissolved in water, fish collagen peptide was weighed and dissolved in the mannitol solution, hydroxyethyl cellulose was weighed and dissolved in the above mannitol-fish collagen peptide solution, and water was added to fix the volume.

**Table 3-1 Formula of an auxiliary material solution**

| Mannitol | 5 g |
|---|---|
| Fish collagen peptide (5000 Da) | 7.5 g |
| Hydroxyethyl cellulose | 0.5 g |
| Add water to fix the volume to | 100 mL |

### 3.1.2 Preparation of a semi-finished solution

The preparation of an allergen stock solution and the mixing of the allergen stock solution with the auxiliary material solution were the same as those in Example 1. The concentration of each allergen extract in the semi-finished solution is as shown in Table 3-2. The semi-finished solution at dose 1 was prepared by proportionally diluting the semi-finished solution at dose 2 with the auxiliary material solution.

**Table 3-2 Concentrations of dust mite extracts in semi-finished solutions**

| Ingredient | Unit | Dose 1 | Dose 2 | Dose 3 |
|---|---|---|---|---|
| *Dermatophagoides pteronyssinus* extract | mg/mL | 0.0825 | 0.25 | 0.5 |
| *Dermatophagoides farinae* extract | mg/mL | 0.33 | 1 | 2 |

### 3.1.3 Filling

The dust mite semi-finished solution prepared in 3.2 was subpackaged into blisters made of an aluminium composite material, 0.3 mL per blister.

### 3.1.4 Lyophilization

The blister plate containing the subpackaged semi-finished solution was placed in a liquid nitrogen quick-freezing tank for 10 min, and the quick-frozen blister plate was placed in a lyophilizer, sublimated once at -25°C and the pressure of 0.15 mbar for a duration of 496 min, and subjected to desorption drying at 20°C and the pressure of 0.05 mbar for a duration of 240 min.

### 3.1.5 Sealing

After the lyophilization was completed, the blister plate was taken out, covered with an all-aluminium film at 200°C, indented, and cut into unit blocks, each block containing 10 blisters, that is, 10 doses.

### 3.2 Quality inspection

### 3.2.1 Appearance

The blister plate film was removed. As shown in Fig. 1, the tablets have no defect or collapse on the surface, and there is no adhesion between the tablets and the blisters.

The tablet has a weight of 31.5± mg and a surface diameter of 13.2±1 mg.

The tablets were graded according to standards in Table 3-3 below. Grading results are shown in Table 3-5 and Table 3-6.

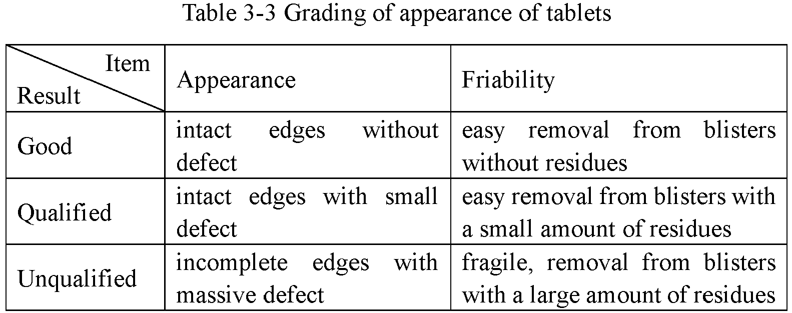

### 3.2.2 Disintegration speed

Referring to the test method of General Rule 0921 of "Chinese Pharmacopoeia IV", the disintegration speed of tablets is shown in Table 3-5 and Table 3-6. The disintegration speed of the tablets prepared in this example is less than 30 s.

### 3.2.3 Uniformity

10 tablets were randomly selected from the prepared tablets, the total activity of each tablet was determined by competitive ELISA, the mean activity and SD of the 10 tablets were calculated, and a tablet deviation was calculated. A tablet activity variance (CV) should be less than 7.5%. Detailed results are as shown in Table 3-4.

**Table 3-4 Uniformity of the active content in each dosage form in Table 3-2**

| Group | Mean active content | SD | CV (%) |
|---|---|---|---|
| Dose 1 | 3212 | 97 | 3 |
| Dose 2 | 10215 | 687 | 6 |
| Dose 3 | 20375 | 999 | 4.9 |

There is no significant difference in results of the 3 doses in the detection of the following indicators, and only the test results of dose 2 are shown.

### 3.2.4 Moisture content detection

The principle of moisture detection by this method was Karl Ficsher's titration method for detecting residual moisture in tablets. As shown in Table 3-5 and Table 3-6, the moisture content of the tablets prepared in this example is less than 5%.

### 3.2.5 Major allergenic protein detection

By using the principle of ELISA double-antibody sandwich, a monoclonal antibody against major allergenic proteins was used as a capture antibody, another monoclonal antibody against major allergenic proteins that was labelled with biotin was used as a detection antibody, and streptavidin-coupled horseradish peroxidase reacted with the biotin-labelled detection antibody, ABTS was used as a reaction substrate for color development, and the content of major allergenic proteins was directly proportional to the color. The content of major allergenic proteins should be in the range of 50-200% of labelled amount.

As shown in Table 3-5 and Table 3-6, the content of major allergenic proteins in the tablet prepared in this example is 95-112%.

### 3.2.6 Total activity detection

The total activity was detected using the principle of ELISA inhibition assay. A nunc chemiluminiscence plate was prepared by coating with an internal reference for the dust mite extract. The dust mite sublingual oral tablet was redissolved in 0.3 mL of purified water, the internal reference for the dust mite extract and the dust mite tablet solution obtained after redissolution were diluted to 5 concentrations in a 2-fold gradient, and the diluted solutions were respectively proportionally mixed with dust mite-specific IgE positive serum and incubated at 37°C for 1 h. The solutions obtained after incubation were transferred to the nunc chemiluminiscence plate coated with the internal reference, and incubated at 37°C for 45 min, after the incubation was completed, the plate was washed 5 times with PBST, 100 uL of horseradish peroxidase-labelled mouse anti-human IgE antibody was added to each well, after the incubation was completed, the plate was washed 5 times, substrates A and B were added, and the plate was read after 5 min. Inhibition curves of the internal reference and the sample to be tested were respectively established, the 50% inhibition rate was calculated, and activity units of the sample to be tested was calculated based on a ratio of the 50% inhibition rate of the sample to be tested to the 50% inhibition rate of the internal reference. The total allergen activity is as shown in Table 3-5 and Table 3-6.

### 3.2.7 Microbial limit

Referring to General Rules 1105, 1106 and 1107 of "Chinese Pharmacopoeia IV", microbial limit detection and microbial counting were carried out, and test results should meet the requirements.

**Table 3-5 Microbial limit test results**

| Item | Result | Standard (CFU) | Standard (CFU) |
|---|---|---|---|
| Total number of aerobic bacteria | 20 | 60 | 100 |
| Total number of molds and yeasts | 8 | 10 | 10 |
| *Escherichia coli* | 0 | 0 | 0 |
| *Salmonella* | 0 | 0 | 0 |
| *Pseudomonas aeruginosa* | 0 | 0 | 0 |
| *Staphylococcus aureus* | 0 | 0 | 0 |

### 3.2.8 Stability test

Accelerated stability: the tablet pack was placed at 40°C and the relative humidity of 75%, and samples were taken out in the 1st month, the 2nd month, the 3rd month, and the 6th month, respectively, to inspect the appearance, moisture content, disintegration time, major allergenic protein content, total activity and microbial limits.

Long-term stability: the tablet pack was placed at 25°C and the relative humidity of 75%, and samples were taken out in the 1st month, the 2nd month, the 3rd month, the 6th month, the 12th month, the 18th month, the 24th month, and the 36th month respectively, to inspect the appearance, moisture content, disintegration time, major allergenic protein content, total activity and microbial limits.

**Table 3-5 Test results of the accelerated stability of dust mite tablets**

| Product | Dust mite tablet | | | | | | |
|---|---|---|---|---|---|---|---|
| Storage condition | 40°C/75% relative humidity | | | | | | |
| Test | Loss of total tablet content (%) | Disintegra tion (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 3.80% | Qualified | 112 | 105 |
| 2 | 0.05% | 10 | Qualified | 4.50% | Qualified | 103 | 108 |
| 3 | 0.04% | 10 | Qualified | 4.70% | Qualified | 98 | 95 |
| 6 | 0.03% | 10 | Qualified | 4.80% | Qualified | 97 | 96 |

**Table 3-6 Test results of the long-term stability of dust mite tablets**

| Product | Dust mite tablet | | | | | | |
|---|---|---|---|---|---|---|---|
| Storage condition | 25°C/75% relative humidity | | | | | | |
| Test | Loss of total tablet content (%) | Disintegra tion (s) | Microbial limit | Moisture content (%) | Appearanc e inspection | Major allergenic protein content (%) | Total allergen activity (%) |
| Sampling (month) | | | | | | | |
| 1 | 0.05% | 10 | Qualified | 3.60% | Qualified | 106 | 110 |
| 2 | 0.04% | 10 | Qualified | 4.20% | Qualified | 103 | 108 |
| 3 | 0.04% | 10 | Qualified | 4.50% | Qualified | 97 | 99 |
| 6 | 0.03% | 10 | Qualified | 4.80% | Qualified | 95 | 96 |
| 12 | 0.05% | 10 | Qualified | 3.60% | Qualified | 106 | 110 |
| 18 | 0.04% | 10 | Qualified | 4.20% | Qualified | 103 | 108 |
| 24 | 0.04% | 10 | Qualified | 4.50% | Qualified | 97 | 99 |
| 36 | 0.03% | 10 | Qualified | 4.80% | Qualified | 95 | 96 |

The dust mite tablets can be stably stored at 40°C and the relative humidity of 75% for 6 months, and the loss of maj or allergenic proteins is 15% only. The dust mite tablets can be stably stored at 25°C and the relative humidity of 75% for 36 months, and the loss of major allergenic proteins is 11% only.

### Example 4 Dust mite allergen preparation with an auxiliary material containing collagen peptide

In this example, only the fish collagen protein of 5000 Da in the formula of the auxiliary material solution in Example 3 was replaced with fish collagen peptide of 3000 Da, and unless otherwise specified, other steps were the same as those in Example 3.

There were also 3 doses in this example, and the concentration of each dose was the same as that in Example 3. There is no significant difference in results of the 3 doses in the detection of various indicators, and only the detection results of various indicators of dose 2 are shown.

The tablets prepared in this example have no defect or collapse on the surface, and there is no adhesion between the tablets and the blisters. The tablet has a weight of 40.5 mg and a surface diameter of 13.2 mm.

Test results of the stability are as shown in Table 4-1 and Table 4-2.

**Table 4-1 Test results of the accelerated stability of dust mite tablets**

| Product | Dust mite tablet | | | | | | |
|---|---|---|---|---|---|---|---|
| Storage condition | 40°C/75% relative humidity | | | | | | |
| Test | Loss of total tablet content (%) | Disintegr ation (s) | Microbial limit | Moisture content (%) | Appearanc e inspection | Major allergenic protein content (%) | Total allergen activity (%) |
| Sampling (month) | | | | | | | |
| 1 | 0.04% | 10 | Qualified | 3.90% | Qualified | 102 | 95 |
| 2 | 0.05% | 10 | Qualified | 4.40% | Qualified | 93 | 103 |
| 3 | 0.06% | 10 | Qualified | 4.60% | Qualified | 108 | 92 |
| 6 | 0.03% | 10 | Qualified | 3.80% | Qualified | 97 | 96 |

**Table 4-3 Test results of the long-term stability of dust mite tablets**

| Product | Dust mite tablet | | | | | | |
|---|---|---|---|---|---|---|---|
| Storage condition | 25°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 4.60% | Qualified | 96 | 110 |
| 2 | 0.04% | 10 | Qualified | 3.20% | Qualified | 103 | 118 |
| 3 | 0.05% | 10 | Qualified | 4.50% | Qualified | 107 | 99 |
| 6 | 0.03% | 10 | Qualified | 4.80% | Qualified | 95 | 96 |
| 12 | 0.04% | 10 | Qualified | 4.60% | Qualified | 105 | 110 |
| 18 | 0.04% | 10 | Qualified | 4.20% | Qualified | 101 | 98 |
| 24 | 0.06% | 10 | Qualified | 3.60% | Qualified | 97 | 109 |
| 36 | 0.03% | 10 | Qualified | 3.80% | Qualified | 98 | 110 |

The dust mite tablets can be stably stored at 40°C and the relative humidity of 75% for 6 months, and the loss of major allergenic proteins is 5% only. The dust mite tablets can be stably stored at 25°C and the relative humidity of 75% for 36 months, and no loss of major allergenic proteins is found.

### Comparative Example 1 Dust mite allergen preparation with an auxiliary material containing low molecular weight maltodextrin

In this comparative example, only the fish collagen peptide of 5000 Da in the formula of the auxiliary material solution in Example 3 was replaced with low molecular weight maltodextrin of 5000 Da, each 100 mL of auxiliary material solution contained 10 g of low molecular weight maltodextrin of 5000 Da, and unless otherwise specified, other steps were the same as those in Example 3.

The tablets prepared in this comparative example have no defect or collapse on the surface, and there is no adhesion between the tablets and the blisters. The tablet has a weight of 40.5 mg and a surface diameter of 13.2 mm.

There is no significant difference in results of the 3 doses in the detection of various indicators, and only the test results of the stability of dose 2 are shown in Table D1-1 and Table D1-2.

**Table D1-1 Test results of the accelerated stability of dust mite tablets**

| Product | Dust mite tablet | | | | | | |
|---|---|---|---|---|---|---|---|
| Storage condition | 40°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.05% | 10 | Qualified | 2.80% | Qualified | 92 | 85 |
| 2 | 0.05% | 10 | Qualified | 4.50% | Qualified | 83 | 78 |
| 3 | 0.04% | 10 | Qualified | 3.70% | Qualified | 78 | 65 |
| 6 | 0.03% | 10 | Qualified | 4.80% | Qualified | 67 | 56 |

**Table D1-2 Test results of the long-term stability of dust mite tablets**

| Product | Dust mite tablet | | | | | | |
|---|---|---|---|---|---|---|---|
| Storage condition | 25°C/75% relative humidity | | | | | | |
| Test | Loss of total tablet content (%) | Disinte gration (s) | Microbial limit | Moisture content (%) | Visual checking | Major allergenic protein content (%) | Total allergen activity (%) |
| Sampling (month) | | | | | | | |
| 1 | 0.05% | 10 | Qualified | 3.70% | Qualified | 86 | 90 |
| 2 | 0.06% | 10 | Qualified | 4.20% | Qualified | 85 | 88 |
| 3 | 0.04% | 10 | Qualified | 4.50% | Qualified | 77 | 79 |
| 6 | 0.04% | 10 | Qualified | 2.80% | Qualified | 57 | 66 |
| 12 | 0.05% | 10 | Qualified | 3.60% | Qualified | 46 | 60 |
| 18 | 0.04% | 10 | Qualified | 4.20% | Qualified | 43 | 58 |
| 24 | 0.04% | 10 | Qualified | 4.50% | Qualified | 37 | 49 |
| 36 | 0.03% | 10 | Qualified | 4.80% | Qualified | 35 | 46 |

This comparative example 1 indicates that compared with the dust mite tablets containing collagen peptide, the dust mite tablets containing low molecular weight maltodextrin can be stably stored at 40°C and the relative humidity of 75% for 1 month only, the content of allergenic proteins decreases rapidly from the 2nd month, and the loss of the allergenic protein content in the 4th month is as high as 25%. The dust mite tablets can be stably stored at 25°C and the relative humidity of 75% for 2 months only, and the loss of the allergenic protein content in the 36th month is as high as 51%.

### Example 5 Artemisia annua pollen allergen preparation

In this example, the allergen in Example 3 was replaced with *Artemisia annua* pollen, and unless otherwise specified, other steps were the same as those in Example 3.

### 5.1 Preparation of an allergen stock solution

Pollen from wild and cultivated *Artemisia annua* was purified and degreased, and added to a disodium hydrogen phosphate-sodium dihydrogen phosphate buffer (200 mM, pH=7.8-8.2) in a feeding ratio of 1: 25, and the solution was extracted at 2-8°C for 20 h. An extract obtained after extraction was centrifuged at 2-8°C and 8000 rpm for 15 min, and a supernatant obtained after centrifugation was filtered with 0.8 µm, 0.45 µm, and 0.22 µm microfiltration membranes in sequence to obtain an *Artemisia annua* pollen extract. The *Artemisia annua* pollen extract obtained after clarification and filtration was subjected to constant-volume ultrafiltration with purified water and a 3 KD membrane to a 5-time volume, and ultra-filtered and concentrated to obtain an *Artemisia annua* pollen stock solution in which the concentration of the *Artemisia annua* pollen extract was 1.6 mg/mL.

### 5.2 Preparation of a semi-finished solution

**Table 5-1 Concentration of an Artemisia annua pollen extract in a semi-finished solution**

| Ingredient | Unit | Dose 1 | Dose 2 | Dose 3 |
|---|---|---|---|---|
| *Artemisia annua* pollen extract | mg/mL | 0.132 | 0.4 | 0.8 |

The 1.6 mg/mL *Artemisia annua* pollen stock solution was uniformly mixed with an auxiliary material solution in a volume ratio of 1: 1 to obtain a semi-finished solution intermediate in which the concentration of the *Artemisia annua* pollen extract was 0.8 mg/mL, the semi-finished solution intermediate in which the concentration of the *Artemisia annua* pollen extract was 0.8 mg/mL was diluted in sequence with the auxiliary material solution to obtain *Artemisia annua* pollen semi-finished solutions in which the concentration of the *Artemisia annua* pollen extract was 0.4 mg/mL or 0.132 mg/mL.

Other steps in the preparation method, quality inspection and the stability test method in this example were the same as those in Example 3.

The tablets prepared in this example have no defect or collapse on the surface, and there is no adhesion between the tablets and the blisters. The tablet has a weight of 40.5 mg and a surface diameter of 13.2 mm.

There is no significant difference in results of the 3 doses in the detection of various indicators, and only the test results of the stability of dose 2 are shown in Table 5-2 and Table 5-3.

**Table 5-2 Test results of the accelerated stability of Artemisia annua pollen tablets**

| Product | *Artemisia annua* pollen tablet | | | | | | |
|---|---|---|---|---|---|---|---|
| Storage condition | 40°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.05% | 10 | Qualified | 2.80% | Qualified | 102 | 105 |
| 2 | 0.05% | 10 | Qualified | 4.50% | Qualified | 103 | 108 |
| 3 | 0.04% | 10 | Qualified | 3.70% | Qualified | 98 | 115 |
| 6 | 0.03% | 10 | Qualified | 4.80% | Qualified | 97 | 96 |

**Table 5-3 Test results of the long-term stability of Artemisia annua pollen tablets**

| Product | *Artemisia annua* pollen tablet | | | | | | |
|---|---|---|---|---|---|---|---|
| Storage condition | 25°C/75% relative humidity | | | | | | |
| Test | Loss of total tablet content (%) | Disinte gration (s) | Microbial limit | Moisture content (%) | Visual checking | Major allergenic protein content (%) | Total allergen activity (%) |
| Sampling (month) | | | | | | | |
| 1 | 0.05% | 10 | Qualified | 3.70% | Qualified | 106 | 100 |
| 2 | 0.06% | 10 | Qualified | 4.20% | Qualified | 105 | 108 |
| 3 | 0.04% | 10 | Qualified | 4.50% | Qualified | 97 | 99 |
| 6 | 0.04% | 10 | Qualified | 2.80% | Qualified | 97 | 106 |
| 12 | 0.05% | 10 | Qualified | 3.60% | Qualified | 106 | 110 |
| 18 | 0.04% | 10 | Qualified | 4.20% | Qualified | 113 | 98 |
| 24 | 0.04% | 10 | Qualified | 4.50% | Qualified | 97 | 99 |
| 36 | 0.03% | 10 | Qualified | 4.80% | Qualified | 95 | 96 |

The *Artemisia annua* pollen tablets can be stably stored at 40°C and the relative humidity of 75% for 6 months, and the loss of major allergenic proteins in the 6th month is 5% only. The *Artemisia annua* pollen tablets can be stably stored at 25°C and the relative humidity of 75% for 36 months, and the loss of major allergenic proteins in the 36th month is 11% only.

### Example 6 Humulus pollen allergen preparation

In this example, only the *"Artemisia annua* pollen extract" in Example 5 was replaced with a *"Humulus* pollen extract", the concentration of the *Humulus* pollen extract in a prepared *Humulus* pollen allergen stock solution was 1.7 mg/mL, and other steps in the preparation method, quality inspection and the stability test were the same as those in Example 5.

According to proportions in Table 6-1, mannitol was weighed and dissolved in water, fish collagen peptide was weighed and dissolved in the mannitol solution, hydroxyethyl cellulose was weighed and dissolved in the above mannitol-fish collagen peptide solution, and water was added to fix the volume.

**Table 6-1 Formula of an auxiliary material solution**

| | |
|---|---|
| Mannitol | 3 g |
| Fish collagen peptide (5000 Da) | 6 g |
| Hydroxyethyl cellulose | 0.5 g |
| Add water to fix the volume to | 100 mL |

**Table 6-2 Concentration of a Humulus pollen extract in a semi-finished solution**

| Ingredient | Unit | Dose 1 | Dose 2 | Dose 3 |
|---|---|---|---|---|
| *Humulus* pollen extract | mg/mL | 0.14025 | 0.425 | 0.85 |

The tablets prepared in this example have no defect or collapse on the surface, and there is no adhesion between the tablets and the blisters. The tablet has a weight of 40.5 mg and a surface diameter of 13.2 mm.

There is no significant difference in results of the 3 doses in the detection of various indicators, and only the test results of the stability of dose 2 are shown in Table 6-3 and Table 6-4.

**Table 6-3 Test results of the accelerated stability of Humulus pollen tablets**

| Product | *Humulus* pollen tablet | | | | | | |
|---|---|---|---|---|---|---|---|
| Storage condition | 40°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 4.80% | Qualified | 102 | 95 |
| 2 | 0.05% | 10 | Qualified | 4.50% | Qualified | 93 | 98 |
| 3 | 0.04% | 10 | Qualified | 2.70% | Qualified | 98 | 95 |
| 6 | 0.03% | 10 | Qualified | 4.50% | Qualified | 97 | 92 |

**Table 6-4 Test results of the long-term stability of Humulus pollen tablets**

| Product | *Humulus* pollen tablet | | | | | | |
|---|---|---|---|---|---|---|---|
| Storage condition | 25°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 2.60% | Qualified | 96 | 102 |
| 2 | 0.04% | 10 | Qualified | 4.20% | Qualified | 93 | 108 |
| 3 | 0.04% | 10 | Qualified | 4.50% | Qualified | 97 | 99 |
| 6 | 0.07% | 10 | Qualified | 3.80% | Qualified | 95 | 96 |
| 12 | 0.05% | 10 | Qualified | 3.60% | Qualified | 106 | 110 |
| 18 | 0.04% | 10 | Qualified | 4.20% | Qualified | 103 | 108 |
| 24 | 0.04% | 10 | Qualified | 3.50% | Qualified | 97 | 94 |
| 36 | 0.05% | 10 | Qualified | 4.80% | Qualified | 95 | 93 |

The *Humulus* pollen tablets can be stably stored at 40°C and the relative humidity of 75% for 6 months, and the loss of major allergenic proteins in the 6th month is 5% only. The *Humulus* pollen tablets can be stably stored at 25°C and the relative humidity of 75% for 36 months, and the loss of major allergenic proteins in the 36th month is 1% only.

### Example 7 Ambrosia artemisiifolia allergen preparation

In this example, only the *"Artemisia annua* pollen extract" in Example 5 was replaced with an *"Ambrosia artemisiifolia* pollen extract", the concentration of the *Ambrosia artemisiifolia* pollen extract in a prepared *Ambrosia artemisiifolia* pollen allergen stock solution was 1.8 mg/mL, and other steps in the preparation method, quality inspection and the stability test were the same as those in Example 5.

According to proportions in Table 7-1, mannitol was weighed and dissolved in water, fish collagen peptide was weighed and dissolved in the mannitol solution, low molecular weight maltodextrin was weighed and added to the mannitol-fish collagen peptide solution, hydroxyethyl cellulose was weighed and dissolved in the above mannitol-fish collagen peptide-low molecular weight maltodextrin solution, and water was added to fix the volume. The auxiliary material solution of this example contained both the low molecular weight maltodextrin and the collagen peptide.

**Table 7-1 Formula of an auxiliary material solution**

| | |
|---|---|
| Mannitol | 3 g |
| Low molecular weight maltodextrin (5000 Da) | 5 g |
| Fish collagen peptide (5000 Da) | 6 g |
| Hydroxyethyl cellulose | 0.5 g |
| Add water to fix the volume to | 100 mL |

**Table 7-2 Formulas of Ambrosia artemisiifolia pollen tablets**

| Ingredient | Unit | Dose 1 | Dose 2 | Dose 3 |
|---|---|---|---|---|
| *Ambrosia artemisiifolia* pollen extract | mg/mL | 0.1485 | 0.45 | 0.9 |

The tablets prepared in this example have no defect or collapse on the surface, and there is no adhesion between the tablets and the blisters. The tablet has a weight of 40.5 mg and a surface diameter of 13.2 mm.

There is no significant difference in results of the 3 doses in the detection of various indicators, and only the test results of the stability of dose 2 are shown in Table 7-3 and Table 7-4.

**Table 7-3 Test results of the accelerated stability of Ambrosia artemisiifolia pollen tablets**

| Product | *Ambrosia artemisiifolia* pollen tablet | | | | | | |
|---|---|---|---|---|---|---|---|
| Storage condition | 40°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 3.90% | Qualified | 102 | 95 |
| 2 | 0.05% | 10 | Qualified | 4.50% | Qualified | 113 | 98 |
| 3 | 0.06% | 10 | Qualified | 3.70% | Qualified | 98 | 105 |
| 6 | 0.03% | 10 | Qualified | 4.80% | Qualified | 95 | 96 |

**Table 7-4 Test results of the long-term stability of Ambrosia artemisiifolia pollen tablets**

| Product | *Ambrosia artemisiifolia* pollen tablet | | | | | | |
|---|---|---|---|---|---|---|---|
| Storage condition | 25°C/75% relative humidity | | | | | | |
| Test | Loss of total tablet content (%) | Disint egrati on (s) | Microbial limit | Moisture content (%) | Appearanc e inspection | Major allergenic protein content (%) | Total allergen activity (%) |
| Sampling (month) | | | | | | | |
| 1 | 0.05% | 10 | Qualified | 3.60% | Qualified | 106 | 110 |
| 2 | 0.04% | 10 | Qualified | 4.20% | Qualified | 103 | 108 |
| 3 | 0.04% | 10 | Qualified | 4.50% | Qualified | 97 | 99 |
| 6 | 0.03% | 10 | Qualified | 4.80% | Qualified | 95 | 96 |
| 12 | 0.05% | 10 | Qualified | 3.60% | Qualified | 106 | 110 |
| 18 | 0.04% | 10 | Qualified | 4.20% | Qualified | 103 | 108 |
| 24 | 0.04% | 10 | Qualified | 4.50% | Qualified | 97 | 99 |
| 36 | 0.03% | 10 | Qualified | 4.80% | Qualified | 95 | 96 |

The *Ambrosia artemisiifolia* pollen tablets can be stably stored at 40°C and the relative humidity of 75% for 6 months, and the loss of major allergenic proteins in the 6th month is 5% only. The *Ambrosia artemisiifolia* pollen tablets can be stably stored at 25°C and the relative humidity of 75% for 36 months, and the loss of major allergenic proteins in the 36th month is 11% only.

The results of the tablets of this example in both the accelerated stability test and the long-term stability test are good.
Comparative Example 2 Tree pollen mixed allergen preparation
Preparation of allergen stock solutions

*Populus* pollen, *Ulmus* pollen, and *Salix* pollen that were collected in the wild were purified and degreased, and respectively added to a disodium hydrogen phosphate-sodium dihydrogen phosphate buffer (200 mM, pH=7.8-8.2) in a feeding ratio of 1: 25, and the solutions were extracted at 2-8°C for 20 h. The extracts obtained after extraction were respectively centrifuged at 2-8°C and 8000 rpm for 15 min, and supernatants obtained after centrifugation were respectively filtered with 0.8 µm, 0.45 µm, and 0.22 µm microfiltration membranes in sequence to obtain a *Populus* pollen extract, an *Ulmus* pollen extract, and a *Salix* pollen extract. The tree pollen extracts obtained after clarification and filtration were respectively subjected to constant-volume ultrafiltration with purified water and a 3 KD membrane to a 5-time volume, and ultra-filtered and concentrated to obtain tree pollen stock solutions in which the concentration of the *Populus* pollen extract, the *Ulmus* pollen extract or the *Salix* pollen extract was 2.1 mg/mL.

The 3 tree pollen stock solutions were mixed in a volume ratio of 1: 1: 1, such that in the mixed solution of the 3 tree pollen stock solutions, the concentrations of the *Populus* pollen extract, the *Ulmus* pollen extract, and the *Salix* pollen extract were diluted to 0.7 mg/mL. Then, the mixed solution was diluted in sequence with an auxiliary material solution to obtain semi-finished solutions at different doses.

### Preparation of an auxiliary material solution

According to proportions in Table D2-1, mannitol was weighed and dissolved in water, low molecular weight maltodextrin was weighed and added to the mannitol solution, hydroxyethyl cellulose was weighed and dissolved in the above mannitol-low molecular weight maltodextrin solution, and water was added to fix the volume.

**Table D2-1 Formula of an auxiliary material solution**

| | |
|---|---|
| Mannitol | 3 g |
| Low molecular weight maltodextrin (5000 Da) | 10 g |
| Hydroxyethyl cellulose | 0.5 g |
| Add water to fix the volume to | 100 mL |

**Table D2-2 Formulas of tree pollen tablets**

| Ingredient | Unit | Dose 1 | Dose 2 | Dose 3 |
|---|---|---|---|---|
| *Populus* pollen extract | mg/mL | 0.05833 | 0.175 | 0.35 |
| *Ulmus* pollen extract | mg/mL | 0.05833 | 0.175 | 0.35 |
| *Salix* pollen extract | mg/mL | 0.05833 | 0.175 | 0.35 |

| | | | | |
|---|---|---|---|---|
| Note: each dose contains the *Populus* pollen extract, the *Ulmus* pollen extract, and the *Salix* pollen extract. | | | | |

Other steps in the preparation method, quality inspection and the stability test method in this example were the same as those in Example 3.

The tablets prepared in this example have no defect or collapse on the surface, and there is no adhesion between the tablets and the blisters. The tablet has a weight of 40.5 mg and a surface diameter of 13.2 mm.

There is no significant difference in results of the 3 doses in the detection of various indicators, and only the test results of the stability of dose 2 are shown in Table D2-3 and Table D2-4.

**Table D2-3 Test results of the accelerated stability of tree pollen tablets**

| Product | Tree pollen tablet | | | | | | |
|---|---|---|---|---|---|---|---|
| Storage condition | 40°C/75% relative humidity | | | | | | |
| Test | Loss of total tablet content (%) | Disinte gration (s) | Microbial limit | Moisture content (%) | Appearanc e inspection | Major allergenic protein content (%) | Total allergen activity (%) |
| Sampling (month) | | | | | | | |
| 1 | 0.06% | 10 | Qualified | 2.80% | Qualified | 92 | 85 |
| 2 | 0.06% | 10 | Qualified | 3.50% | Qualified | 83 | 78 |
| 3 | 0.04% | 10 | Qualified | 4.70% | Qualified | 68 | 75 |
| 6 | 0.05% | 10 | Qualified | 4.80% | Qualified | 57 | 56 |

**Table D2-4 Test results of the long-term stability of tree pollen tablets**

| Product | Tree pollen tablet | | | | | | |
|---|---|---|---|---|---|---|---|
| Storage condition | 25°C/75% relative humidity | | | | | | |
| Test | Loss of total tablet content (%) | Disint egrati on (s) | Microbial limit | Moisture content (%) | Appearanc e inspection | Major allergenic protein content (%) | Total allergen activity (%) |
| Sampling (month) | | | | | | | |
| 1 | 0.05% | 10 | Qualified | 3.60% | Qualified | 86 | 85 |
| 2 | 0.06% | 10 | Qualified | 4.20% | Qualified | 83 | 80 |
| 3 | 0.04% | 10 | Qualified | 4.50% | Qualified | 77 | 79 |
| 6 | 0.04% | 10 | Qualified | 4.80% | Qualified | 75 | 67 |
| 12 | 0.05% | 10 | Qualified | 3.60% | Qualified | 66 | 60 |
| 18 | 0.05% | 10 | Qualified | 4.20% | Qualified | 63 | 68 |
| 24 | 0.04% | 10 | Qualified | 4.50% | Qualified | 57 | 62 |
| 36 | 0.03% | 10 | Qualified | 4.80% | Qualified | 55 | 46 |

The tree pollen tablets can be stably stored at 40°C and the relative humidity of 75% for 1 month only, the content of allergenic proteins decreases rapidly from the 2nd month, and the loss of the allergenic protein content in the 4th month is as high as 35%. The tree pollen tablets can be stably stored at 25°C and the relative humidity of 75% for 2 months only, and the loss of the major allergenic protein content in the 36th month is as high as 31%.

It can be seen from Examples 4 to 7 and Comparative Examples 1 and 2 that the collagen peptide serving as an auxiliary material can bring good stability to allergen tablets. It can be seen from the results of Example 2 that unlike the formula in which the fish gelatin or corn starch is used as an auxiliary material, the auxiliary material containing the collagen peptide does not introduce allergens and is safe.

## Claims

1. Application of collagen peptide in the preparation of an allergen preparation auxiliary material.

2. An auxiliary material composition, comprising collagen peptide.

3. The auxiliary material composition according to claim 2, further comprising mannitol;
preferably, further comprising cellulose;
preferably, the cellulose being selected from one or two of hydroxyethyl cellulose and hydroxypropyl methyl cellulose;
preferably, the auxiliary material composition containing the following substances in parts by weight:
| | |
|---|---|
| mannitol | 2-8 parts, preferably 2-5 parts; |
| collagen peptide | 2-10 parts, preferably 3-8 parts; and |
| cellulose | 0.2-2 parts, preferably 0.4-1 part; |
preferably, the auxiliary material composition being a solution; and
preferably, the concentration of each substance in the auxiliary material composition solution being as follows:
| | |
|---|---|
| mannitol | 20-80 g/L, preferably 20-50 g/L; |
| collagen peptide | 20-100 g/L, preferably 30-80 g/L; and |
| cellulose | 2-20 g/L, preferably 4-10 g/L. |

4. The application according to claim 1, or the composition according to claim 2 or 3, wherein the collagen peptide is selected from one or more of fish collagen peptide, bovine bone collagen peptide, bovine skin collagen peptide, and pig skin collagen peptide;
preferably, the collagen peptide is selected from one or two of fish collagen peptide and bovine bone collagen peptide;
preferably, the collagen peptide is fish collagen peptide;
preferably, the fish collagen peptide is cold water fish collagen peptide; and
preferably, the cold water fish is selected from *Oncorhynchus, Thunnus albacares, Gadus, Anguilla, Pleuronichthys cornutus, Acipenser sinensis, Perca fluviatilis,* and *Oreochromis mossambicus.*

5. The application according to claim 1, or the composition according to claim 2 or 3, wherein the collagen peptide has a molecular weight of 3000-10000 Da;
preferably, the collagen peptide has a molecular weight of 3000-9000 Da;
preferably, the collagen peptide has a molecular weight of 3000-8000 Da;
preferably, the collagen peptide has a molecular weight of 3000-7000 Da;
preferably, the collagen peptide has a molecular weight of 3000-6000 Da; and
preferably, the collagen peptide has a molecular weight of 3000-5000 Da.

6. Application of the auxiliary material composition according to claim 2 or 3 in the preparation of an allergen preparation.

7. An allergen preparation semi-finished product, wherein an auxiliary material of the semi-finished product contains collagen peptide;
preferably, the allergen semi-finished product contains the auxiliary material composition solution according to claim 3; and
preferably, the allergen semi-finished product contains 0.05-2.5 mg, preferably 0.25-1 mg, more preferably 5-50 ug, of allergen extract.

8. An allergen preparation, wherein an auxiliary material of the preparation contains collagen peptide;
preferably, a dosage form of the allergen preparation is selected from lyophilized powder, injection, tablet, capsule, drop, and patch;
preferably, the dosage form of the allergen preparation is a tablet, more preferably a sublingual oral tablet;
preferably, after the tablet is stored at 40°C and the relative humidity of 75% for 6 months, the loss of the allergen content in the table is less than 25% of labelled content; and further preferably, the loss of the allergen content in the tablet is less than 15% of the labelled content; and
preferably, after the tablet is stored at 25°C and the relative humidity of 75% for 36 months, the loss of the allergen content in the dosage form is less than 25% of the labelled content; and further preferably, the loss of the allergen content in the tablet is less than 15% of the labelled content.

9. The application according to claim 6, or the allergen preparation semi-finished product according to claim 7, or the allergen preparation according to claim 8, wherein the allergen is selected from one or more of a house dust mite allergen, a pollen allergen, a mold allergen, an insect allergen, an animal dander allergen, and a food allergen;
preferably, the house dust mite allergen is selected from one or two of *Dermatophagoides pteronyssinus* and *Dermatophagoides farinae*;
preferably, the house dust mite allergen is one or more of allergenic proteins Derp1, Derf1, Derp2, and Derf2 that are separated from natural extracts of *Dermatophagoides pteronyssinus* and *Dermatophagoides farinae* or are recombinantly expressed;
preferably, the pollen allergen is selected from one or more of a weed pollen allergen and a tree pollen allergen;
preferably, the weed pollen allergen is selected from one or more of *Artemisia sieversiana* pollen, *Artemisia vulgaris* pollen, *Artemisia annua* pollen, *Ambrosia artemisiifolia* pollen, *Ambrosia trifida* pollen, *Humulus* pollen, *Chenopodium album* pollen, *Xanthium strumarium* pollen, and *Bassia scoparia* pollen;
preferably, the tree pollen allergen is selected from one or more of *Populus* pollen, *Salix* pollen, *Ulmus* pollen, *Juniperus chinensis* pollen, *Fraxinus pennsylvanica* pollen, *Platanus* pollen, *Betula* pollen, *Cryptomeria* pollen, and *Acer* pollen; and more preferably, the *Platanus* pollen is selected from one or more of *Platanus orientalis* pollen and *Platanus acerifolia* pollen; and
preferably, the animal dander allergen is selected from one or two of cat dander and dog dander.

10. The application according to claim 6, or the allergen preparation semi-finished product according to claim 7, or the allergen preparation according to claim 8, wherein the allergy is an IgE-mediated allergy;
preferably, the allergy is a specific IgE-mediated allergy; and
preferably, the allergy comprises one or more of allergic urticaria, allergic conjunctivitis, allergic rhinitis, and allergic asthma.

11. A preparation method of an allergen preparation, comprising the following steps:
filling a semi-finished solution of an auxiliary material solution containing collagen peptide and an allergen stock solution into blisters, and transferring the blisters to a lyophilizer for lyophilization; the concentration of each substance in the auxiliary material solution being as follows:
| | |
|---|---|
| mannitol | 20-80 g/L, preferably 20-50 g/L; |
| collagen peptide | 20-100 g/L, preferably 30-80 g/L; and |
| cellulose | 2-20 g/L, preferably 4-10 g/L; |
preferably, the semi-finished solution being prepared by the following method: mixing the auxiliary material solution containing collagen peptide with the allergen stock solution containing an allergen extract;
preferably, the preparation method comprising the following steps:
1) preparation of an allergen stock solution: purifying and degreasing an allergen, adding to a disodium hydrogen phosphate-sodium dihydrogen phosphate buffer (50-200 mM, PH=7.8-8.2) in a feeding ratio of 1: (25-50), extracting at 2-8°C for 4-24 h, centrifuging an extract obtained after extraction at 2-8°C and 6000-10000 rpm for 2-50 min, collecting and filtering a supernatant obtained after centrifugation to obtain an allergen extract, performing constant-volume ultrafiltration on the allergen extract obtained after clarification and filtration with purified water and a 3-5 KD membrane, and ultra-filtering and concentrating to the concentration of the allergen extract being not less than 0.75 mg/mL;
2) preparation of an auxiliary material solution: weighing and dissolving mannitol in water, weighing and adding collagen peptide to the mannitol solution, weighing and dissolving cellulose in the above mannitol-collagen peptide solution, and adding water to fix the volume;
3) preparation of an allergen semi-finished product: uniformly mixing the allergen stock solution prepared at step 1) with the auxiliary material solution prepared at step 2) in a volume ratio;
4) filling: filling the semi-finished solution prepared at step 3) into blisters;
5) lyophilization: transferring the blisters prepared at step 4) to a lyophilizer for lyophilization, preferably, placing the blisters prepared at step 4) in a liquid nitrogen tunnel or a liquid nitrogen freezer for 5-10 min, and transferring to a lyophilizer for lyophilization; and
6) sealing: after the lyophilization at step 5) is completed, taking out the blisters, covering with a film at 160-200°C, indenting, and cutting into unit blocks.

12. The preparation method according to claim 11, wherein at step 1), the supernatant obtained after centrifugation is filtered with a microfiltration membrane;
preferably, the supernatant obtained after centrifugation is filtered with 0.8 µm, 0.45 µm, and 0.22 µm microfiltration membranes in sequence;
preferably, the allergen extract obtained after clarification and filtration is subjected to constant-volume ultrafiltration with purified water and the 3-5 KD membrane to a 5-time volume; and
preferably, at step 4), 0.2-0.35 mL of allergen semi-finished solution prepared at step 3) is subpackaged into each blister.

13. An allergen preparation prepared by the preparation method according to claim 11 or 12, wherein a dosage form of the allergen preparation is selected from lyophilized powder, injection, tablet, capsule, drop, and patch;
preferably, the dosage form of the allergen preparation is a tablet, more preferably a sublingual oral tablet;
preferably, after the tablet is stored at 40°C and the relative humidity of 75% for 6 months, the loss of the allergen content in the table is less than 25% of labelled content;
and further preferably, the loss of the allergen content in the tablet is less than 15% of the labelled content; and
preferably, after the tablet is stored at 25°C and the relative humidity of 75% for 36 months, the loss of the allergen content in the dosage form is less than 25% of the labelled content; and further preferably, the loss of the allergen content in the tablet is less than 15% of the labelled content.
